# EUROPEAN PATENT APPLICATION

(11) **EP 1 279 740 A1**
(43) Date of publication of application: **29.01.2003**
(21) Application number: 01870167.2
(22) Date of filing: 26.07.2001
(51) Int. Cl.: C12N 15/86, A61K 48/00, C12N 5/16

(54) **Recombinant vector derived from adeno-associated virus for gene therapy**

(71) Applicant: VRIJE UNIVERSITEIT BRUSSEL, 1050 Brussel (BE)
(72) Inventor: De Greve, Jacques, B-1700 Dilbeek (BE); Teugels, Erik, B-9255 Buggenhout (BE); Neyns, Bart, B-1730 Asse (Mollem) (BE); Zeinoun, Ziad, B-1090 Brussels (BE); Vermeij, Joanna, B-1080 Brussels (BE)
(74) Representative: Van Malderen, Joelle

(57) **Abstract**

The present invention is related to a recombinant adeno-associated viral construct comprising at least:
- a first terminal repeat of an Adeno Associated Virus
- a strong heterologous promoter
- an heterologous DNA with at least 90% homology to the gene encoding for a *constitutively activated TGF-β1 peptide,* said gene being under the control of said promoter
- a polyadenylation signal
- a second terminal repeat of an Adeno Associated Virus

## Description

### Field of the invention

The present invention is related to a new vector having the ability to inhibit cancer cell growth, especially epithelial cancer cell growth, to a host cell containing said vector, to a pharmaceutical composition containing said vector or said cell for the treatment of cancers, especially epithelial cancers, by gene therapy.

### Background of the invention and state of the art

Cancer is one of the most frequent causes of death of both males and females. Many cancers are difficult to cure with current treatment methods. One such examples is ovarian cancer. In over eighty percent of cases, ovarian tumors are of the epithelial type and originate from the cellular surface epithelium overlying the ovaries.

In many cases these tumors are extremely difficult to treat, especially in advanced cancer with metastases. Currently available therapies include surgery, radiation therapy, chemotherapy, radioimmunotherapy, cytokine treatment and hyperthermia. All these treatment modalities have important limitations and disadvantages. For example, surgery can only be performed on localised accessible tumors, radiation and chemotherapy are associated with both acute and latent toxicity; radioimmunotherapy and hyperthermia have limited application and effectivity. Moreover, most of these techniques are not discriminating techniques and destroy not only tumor cells but also normal cells, with various side-effects on patients. However, in general, the efficiency of said therapies and their combinations is still unsatisfactory.

More recently, gene therapy has been proposed as a novel approach to treat malignancies. Gene therapy consists of correcting a deficiency or an abnormality or of ensuring the expression of a protein of therapeutic interest, by introducing genetic information into the cell or organ concerned. This genetic information can be introduced either *in vitro* into a cell extracted from the organ, with the modified cell then being introduced into the organism, or directly *in vivo* in the appropriate tissue.

The introduction of a molecule carrying genetic information can be achieved by various methods known in the art. Preferred methods use gene delivery vehicles derived from viruses, including adenoviruses, retroviruses, vaccina viruses and adeno associated viruses. Among these viruses, adeno-associated viruses (AAV) offer certain attractive properties for gene therapy. The wild type AAVs are DNA viruses of relatively small size which integrate, in a stable and site-specific manner, into the genome of the cells they infect. They are able to infect a wide spectrum of cell species and tissue types, without having any significant effect on cell growth, on cell morphology or on cell differentiation. In particular, they can express a transgene to high levels in epithelial cells. Moreover, so far no human disease has been found to be asssociated with AAV infection.

Examples of the use of vectors derived from AAVs for transferring genes *in vitro* and *in vivo* have been described in the literature: see in particular; documents WO 91/18088, WO 93/09239, US Patent. No 4,797,368, US Patent No. 5,139,941 and EP 488 528.

However, the genetics of cancer and the molecular mechanisms operating in cells for maintaining the integrity of tissues are so complicated, that the potential ways for treating cancer are numerous and it is impossible to predict *a priori* the efficacy of a potential way of treatment. Different treatment strategies are currently under investigation. For example, one treatment strategy involves the enhancement of immunogenicity of tumor cells *in vivo* by the introduction of cytokine genes. Another treatment involves the introduction of genes that encode enzymes capable of conferring to the tumor cells sensitivity to chemotherapeutic agents. Another strategy involves the delivery of normal tumor suppressor genes and/or inhibitors of activated oncogenes into tumor cells.

Considering the importance of the challenge for obtaining an efficient gene transfer system, efforts to build up new constructs to be tested *in vivo* are always appreciated.

Transforming Growth Factor beta (TGF-β) represents a large family of secreted polypeptides called TGF-b superfamily, including TGF-β1, TGF-β2, TGF-β3, TGF-β4, TGF-β5, inhibins, activins, Mullarian Inhibiting Substance (MIS), Bone Morphogenic Protein (BMP) and Decapentaglegic Product (DPP). TGF-β1 has a wide range of biological activities in different cell types and different organisms and acts as an inhibitor of cell proliferation, an inducer of differentiation and an immunosuppressor. In particular, TGF-β1 induces an autocrine growth suppression in many epithelial cells. The loss of the autocrine growth suppressive circuit of TGF-β1 may be an important step in cancer formation since many cancer cells lose their sensitivity to TGF-β1 growth inhibition and many others retain the responsiveness but do not produce enough active peptide to sustain the autocrine growth suppressive loop (Zeinoun *et al., Anticancer Research* **19,** 413-420 (1999)). For the latter kind of cancer cell types, it would be interesting to introduce a vector comprising the gene encoding the bioactive peptide. Hence, such a vector has never been proposed.

### Aims of the invention

The present invention aims to provide a vector which does not present the drawbacks of the products of the state of the art.

The present invention also aims to provide new vector having the ability to inhibit epithelial cancer cell growth when transferred therein.

Another aim of the present invention is to provide a pharmaceutical composition containing said vector or said cell for use in genetic therapy.

### Summary of the invention

The present invention is related to a recombinant adeno-associated viral construct comprising at least:
- a first terminal repeat of an Adeno Associated Virus
- a strong heterologous promoter
- donor-acceptor splicing signals of SV40 16S/19S protein
- an heterologous DNA corresponding to the gene encoding for a *constitutively activated TGF-β1 peptide,* said gene being under the control of said promoter the polydenylation signal of the bovine growth hormone gene
- a second terminal repeat of an Adeno Associated Virus.

With construct is meant a genetic construct, which can be comprised in a plasmid and/or in a recombinant viral particle.

The recombinant adeno-associated viral construct may further comprise nucleotidic sequences encoding suitable regulatory elements so as to effect expression of the *constitutively activated TGF-β1 peptide* in a suitable host cell.

The present invention is also related to a pharmaceutical composition comprising said recombinant adeno-associated viral construct alone or with a pharmaceutically acceptable carrier. Preferably, the recombinant adeno associated viral construct is comprised in a recombinant viral particle.

The present invention is also related to a method for inhibiting the proliferation of cells, comprising at least the step of transferring a sufficient amount of the recombinant adeno-associated viral construct according to the invention into said cells.

Preferably, said cells are epithelial cells.

Preferably, said epithelial cells are mammary epithelial cells, ovarian epithelial cells or lung epithelial cells.

Preferably, said cells are cancer cells.

Preferably, said cancer cells are selected from the group consisting of human melanoma cells, human mammary tumor cells, human ovarian tumor cells , human lung tumor cells, human sarcoma cells and carcinoma cells.

The present invention is also related to the use of a sufficient amount of the pharmaceutical composition according to the invention for the preparation of a medicament in the treatment and/or the prevention of cancers.

The present invention is related to a non-human animal, genetically modified by the recombinant adeno-associated viral construct according to the invention.

### Brief description of the drawings

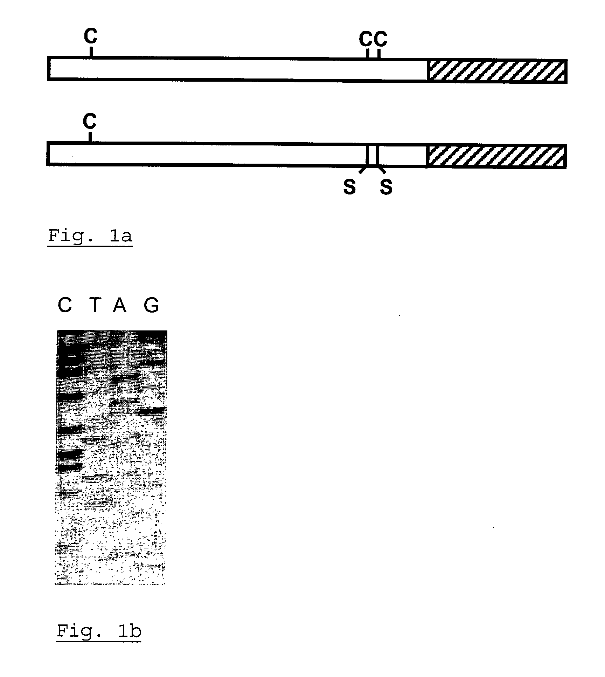

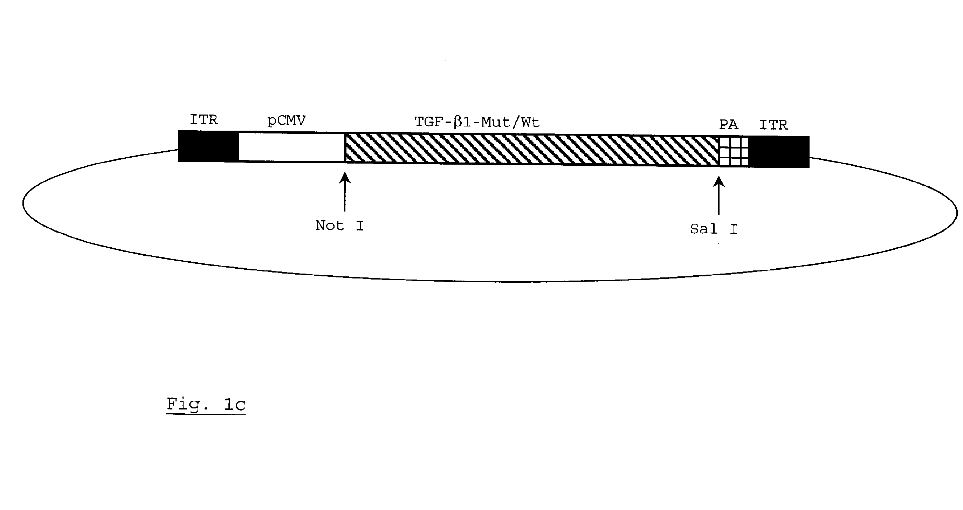

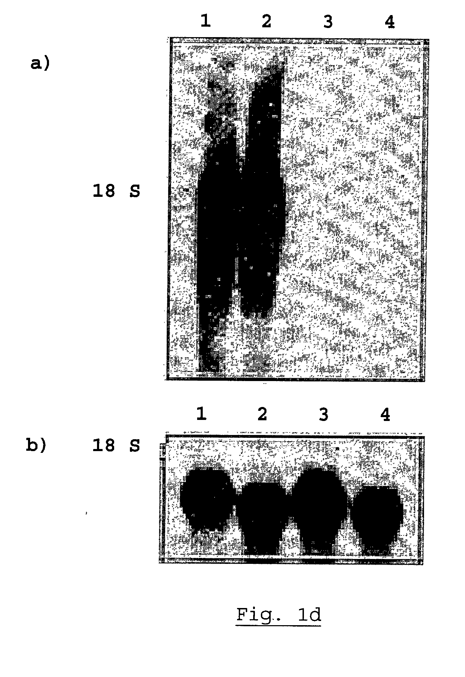

Fig.1 a is a schematic representation of the TGF-β1 propeptide with the two cysteines (CC) at position 223 and 225 (TGF-β1 wild type) replaced with two serines (SS - Mut TGF-β1). Fig.1 b represents the sequence of mutant TGF-β1 DNA in the modified region. Fig.1 c shows TGF-β1 cloned into an AAV expression plasmid. ITR: inverted terminal repeats (145 nucleotides). (pCMV: cytomegalovirus promoter (620 nucleotides), TGF-β1 Wt/Mut: transforming growth factor beta 1 open reading frame, either the wild type (Wt) or the mutant (Mut), PA: polyadenilation signal (230 nucleotides)). Fig.1 d represents a Northern blot of a) TGF-β1 mRNA and b) GAPDH mRNA. The four lanes contain RNA extracted from 293 cells transfected respectively with: 1) rAAV/mutant TGF-β1 plasmid 2) rAAV/wild type TGF-β1 plasmid 3) rAAV/GFP plasmid 4) Non transfected cells. Fig. 1 e outlines the strategy followed to obtain the plasmid encoding the genome of the recAAV-TGF-β1 Mut or Wt virus.

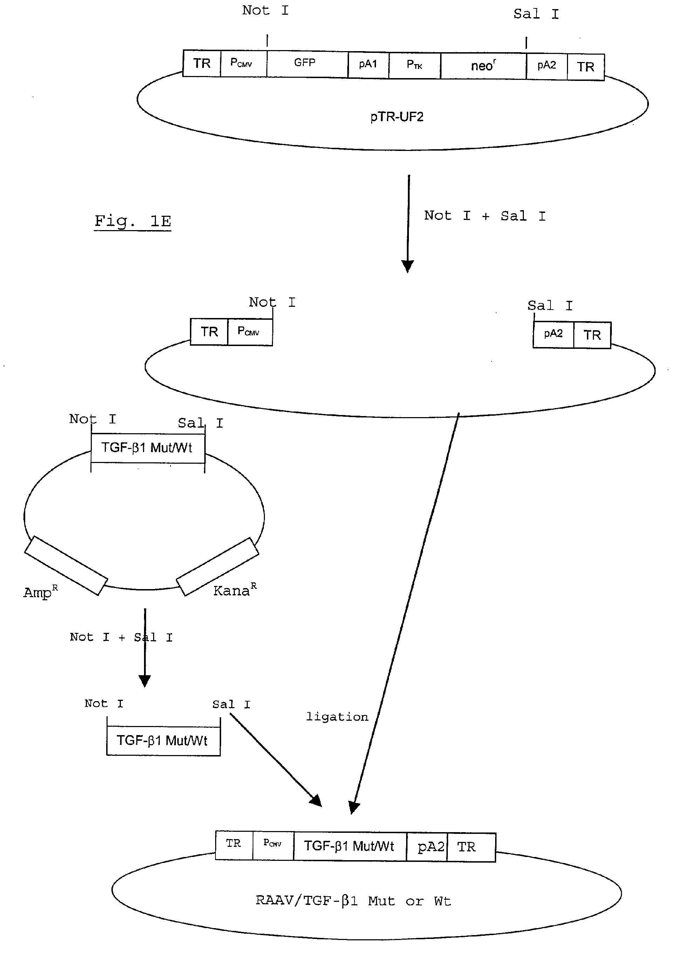

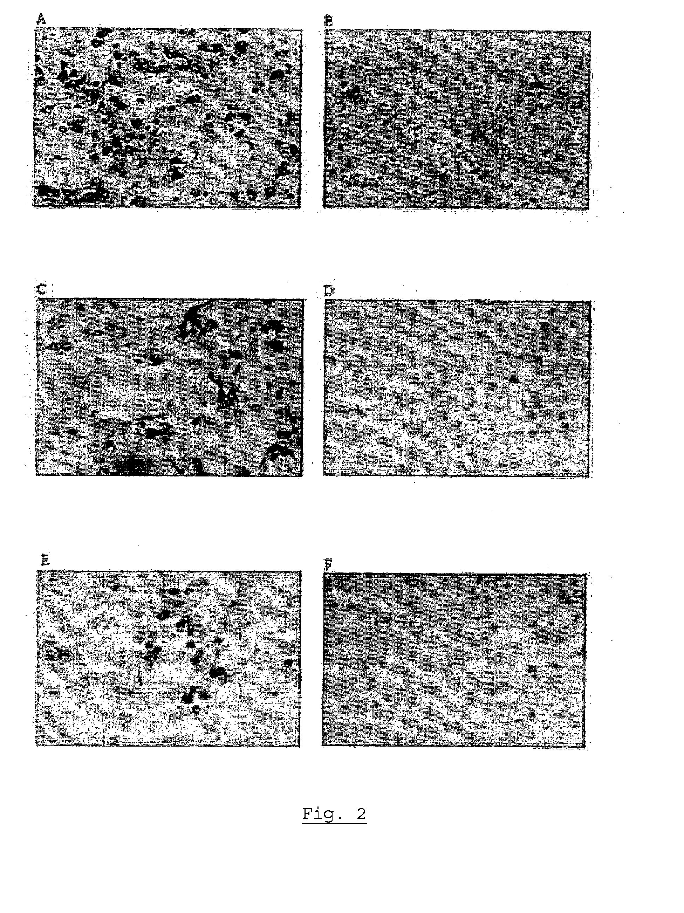

Fig. 2 represents immunostaining with TGF-β1 pAb of cells transfected or infected with TGF-β1 constructs. The 293 cells were transfected with TGF-β1 Mut cDNA (a) or mock transfected (b). The HeLa and OVCAR cells were infected with rAAV/TGF-β1 Mut viral vectors (c, e respectively) or mock infected (d, f respectively).

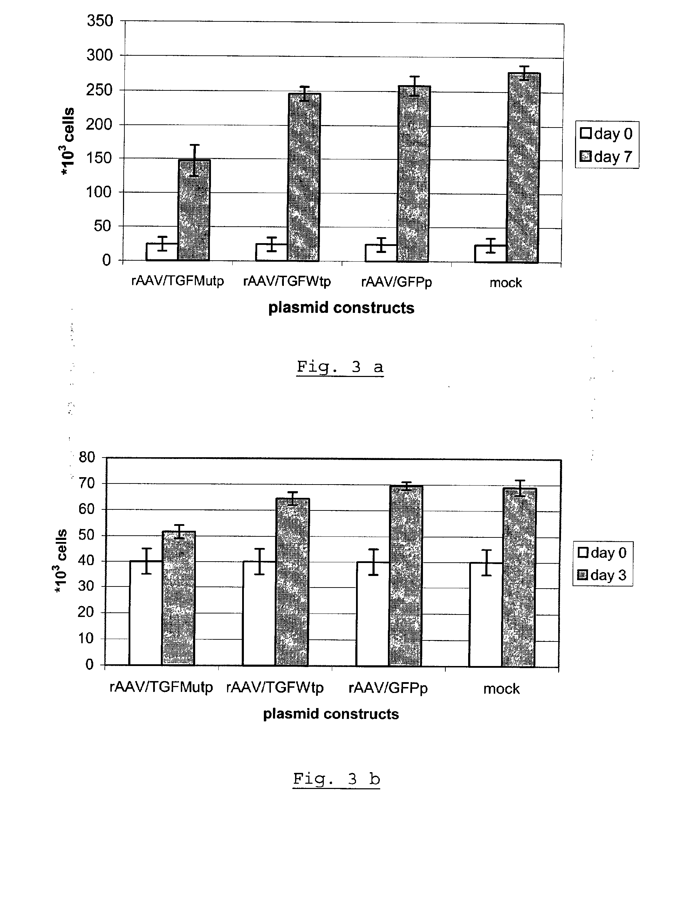

Fig. 3 shows AZ364 (a) and OVCAR (b) cells that were transfected with the rAAV plasmid constructs and cell numbers were determined after 7 and 3 days of incubation respectively.

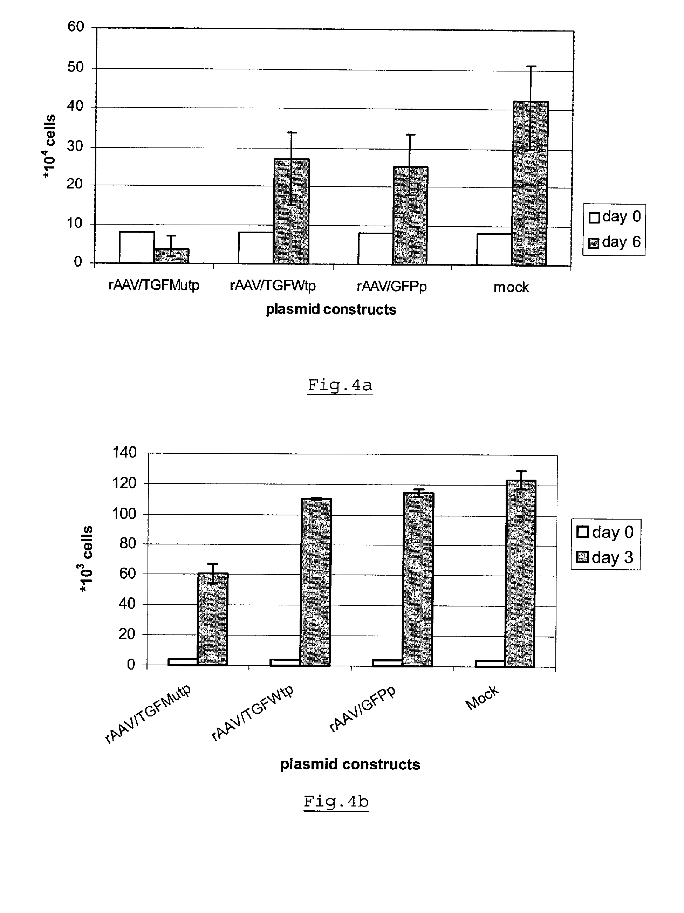

Fig.4 a depicts supernatants from 293 cells previously transfected with the expression plasmids coding for TGF-β1 and GFP, applied onto CCL-64 cells. Cell numbers of CCL-64 cells are shown at Day 0 (supernatant application) and at Day 6. Fig. 4 b shows the effect of supernatants from OVCAR cells transfected with rAAV expression plasmids on CCL-64 cells.

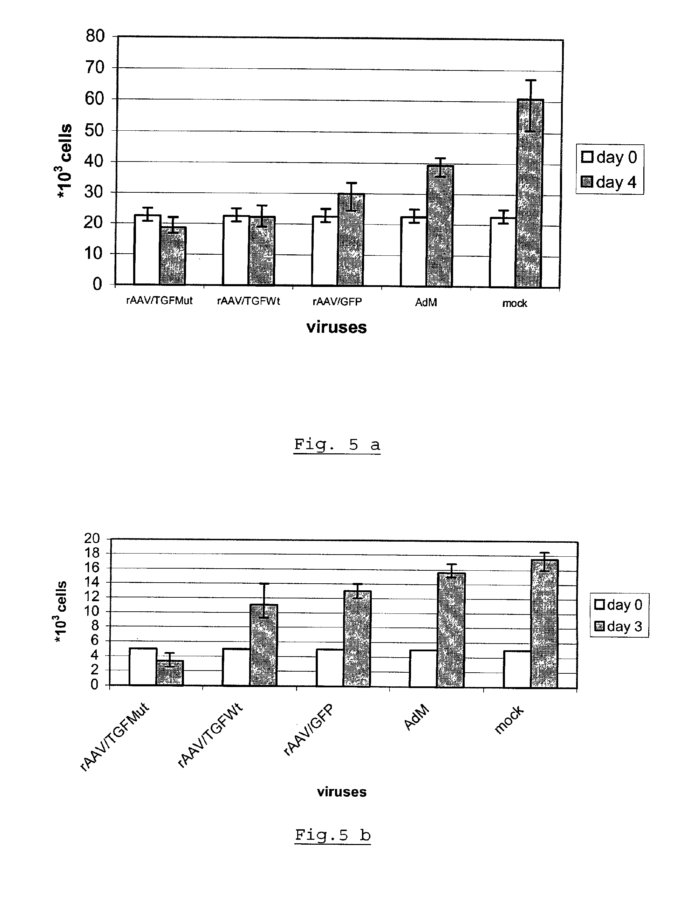

Fig.5 shows a) OVCAR cells were infected with rAAV/TGF-β Mut and Wt and rAAV/GFP viral constructs and controls and cell numbers were determined after 4 days of incubation and b) OVCAR cells were infected with rAAV/TGF-β Mut, Wt and rAAV/GFP viral constructs and controls. Supernatants were collected after 3 days of incubation and transferred onto CCL-64 cells. CCL-64 cell numbers were determined after 3 days of incubation in OVCAR's conditioned media.

### Detailed description of a preferred embodiment according to the invention

The present invention will be further clarified using examples that do not limit the scope of protection as defined by the claims.

### List of abbreviations:

TGF-β1: transforming growth factor beta.
Mut: mutant.
Wt: wild type.
rAAV: recombinant adeno-associated virus.
GFP: green fluorescent protein.
Ad: adenovirus.
AdM: adenovirus mutant.
p: plasmid.

### Introduction

The transforming growth factor beta family of polypeptide growth factors is involved in cell growth regulation, tissue remodelling and immune suppression. The prototype of the family is TGF-β1, secreted mainly as a biologically latent homodimer that can be activated in the extracellular environment. Normal epithelial cells produce TGF-β1 that acts in an autocrine manner to inhibit proliferation. The disruption of this TGF-β1 autocrine growth suppressive circuit seems to play an important part in the progression towards a malignant phenotype. It has been demonstrated that some ovarian cancer cell lines are no longer sensitive to TGF-β1 growth suppressive effect probably because of mutations in the growth related signal transduction pathway. Abnormalities can range from receptors' mutations to post-receptors signaling defects in ovarian cancer cells.

On the other hand, other ovarian cancer cell lines are still sensitive to exogenous TGF-β1 but do not secrete sufficient amounts of the peptide to suppress their own growth. In the same work, we determined the minimal amount of added exogenous TGF-β1 necessary to inhibit their growth. Adding exogenous TGF-β1 is not a feasible approach for *in vivo* correction of this deficit.

### Material and Methods

### Cell lines

The human epithelial ovarian cancer cell line OVCAR was cultured in RPMI 1640 supplemented with 20% fetal bovine serum (FBS) and 10mg/ml bovine insulin. The mink lung epithelial cell line CCL-64, HeLa human cervical carcinoma cell line and 293 E1A-transformed human embryonic epithelial kidney cell line were cultured in DMEM supplemented with 10% fetal bovine serum. AZ364 was obtained from ascites in a patient with a serous cystadenocarcinoma at the AZ-VUB and cultured in RPMI and 10% FBS. Penicillin/streptomycin was added to all media. All cell lines were incubated at 37 °C and 5% CO2.

### PCR amplification and Mutagenesis (Fig. 1 a, b)

The TGF-β1 ORF was PCR amplified with introduction of restriction sites for Not1 and Sal1 at the 5' and 3' ends of forward and reverse primers respectively; forward primer: 5' ATATGCGGCCGCCTGTTCGCGCTCTCGGA 3' and reverse primer: 5' ATATGTCGACTCAGCTGCACTTGCAGGA 3'. The PCR cycles' conditions were as follows: 1st cycle is 1 min. at 94 °C; the 2nd through 5th cycles are 1 min. at 94 °C, 1 min. at 54 °C, 1 min. at 72 °C; the 3rd through 25th cycles are 1 min at 94 °C, 1 min. at 65 °C, 1 min at 7 °C; the final cycle is 10 min at 72 °C.

The PCR product was purified and cloned in a TA cloning vector (ampicillin resistance). Alkaline denaturation of the ds DNA template was performed followed by annealing of the mutagenic oligonucleotide and the selection oligonucleotide; mutagenic oligonucleotide: 5' CCTTAGCGCCCACAGCTCCTCTGACAGCA 3' T4 DNA polymerase and T4 DNA ligase synthesized the mutant strand and the construct was transformed into the BMH71-18 bacterial cells. Transformed bacteria were grown in an antibiotic selection mix. The plasmid DNA was then isolated and transformed again into JM109 bacteria. Mutants were selected on agar plates with antibiotic selection mix (Promega: GeneEditor *in vitro* Site-Directed Mutagenesis System). Mutant plasmid DNA was extracted from the bacteria. All plasmid extractions were based on QIAGEN plasmid extraction kits.

### Sequence analysis

The cloned fragment was sequenced using the 70770 sequenase version 2.0 sequencing kit (Amersham),using the ddNTP method according to Sanger et al., using ³⁵S labeled dATPs.

The sequence obtained is CCCACAGCTCCTCTGAC. The Wt sequence is CCCACTGCTGGTGTGAC. Mutations are underlined.

### Cloning into AAV plasmid (Fig. 1 e)

pTR-UF2 plasmid was digested with Not1 and Sall restriction enzymes (5 unit/µg) and purified (QIAGEN gel extraction kit). TGFβ1Wt/Mut (wild type or mutant) was cloned into the digested pTRUF2. TGFβ1Wt/Mut PCR products were purified (Boeringer Mannheim) and left to ligate overnight at 16 °C into the above mentioned digested vector (T4 DNA ligase; Boeringer Mannheim). The ligation mixtures were then electroporated into JC8III bacteria. Candidate colonies were tested by PCR for the presence of TGFβ1 DNA. Positive colonies were grown and the correspondent plasmids (rAAV/TGF-β1Mut and TGF-β1Wt) were extracted (QIAGEN plasmid extraction Kit).

A deposit has been made according to the Budapest Treaty for the plasmid rAAV/TGF-β1Mut (pTR-TGFbetalMut) under deposit number LMBP 4281 at the BCCM/LMBP Plasmid Collection, Laboratorium voor Moleculaire Biologie, Ledeganckstraat 35, B-9000 Gent (Belgium).

### Transfection of 293 cells

Embryonic Kidney 293 cells were grown in DMEM 10 % FCS. Eight µg of DNA (rAAV/TGF-β1Mut or TGF-β1Wt) and 50 µl of lipofectamine were mixed and added to the cells in a 10-cm culture dish. After 5 hours of incubation the media were refreshed. Three days after transfection the supernatants were collected for growth inhibition assay. Cells were harvested for RNA extraction.

### Northern blot and Hybridization

RNA was extracted from transfected 293 cells (Total RNA extraction kit from QIAGEN). Ten µg/lane of RNA were electrophoresed in an agarose gel and transferred to a nylon membrane. Membranes were hybridized with a ³²P dCTP labeled probes (TGFβ1 and GAPDH probes) at 42°C overnight.

### Growth inhibition assay

Mink lung epithelial cells CCL-64 were plated in 24 well plate in DMEM +10 % FCS at a concentration of 8x10³ cells/well. Conditioned media from transfected 293 and OVCAR cells were then transferred onto CCL-64 cells and left to incubate for three or six days (when controls reached confluence). The CCL-64 cells were then harvested and counted under a light microscope using a hematocytometer.

### Packaging AAV vectors

293 cells were plated and grown in 10-cm culture plates to 80% confluence in DMEM +10% FCS. 2.6 µg/plate of rAAV plasmid along with 5.3 µg/plate of the helper plasmid pIM45 (rep and cap genes) were transfected into the cells by the liposomes' transfection method (50 µl of lipofectamine/plate) in serum free medium. After 5 hours, Adenovirus Ad5 was added at an MOI of one or more in DMEM +2% FCS and left to incubate for a 2 hour period. In the end the medium was changed back to DMEM +10% FCS. After 72 hours, the supernatant was collected along with the cells and subjected to 3 cycles of freeze-thaw. The lysate was incubated at 56°C for 30 min. to inactivate Ad5. The lysate was centrifuged at 3000 rpm for 15 min. and the supernatant was stored in aliquots at -80 °C (13).

### Amplifying rAAV particles

Recombinant AAV particles were amplified by co-infection of HeLa cells (5x10⁶cells/10cm plate) with equal amounts of rAAV and wtAAV infectious particles (10³ /10³) followed by adenovirus infection at a MOI of 1. After 3 days the lysate was collected and treated in a similar way as described for the co-transfection stock. The rAAV particles (amplified stock) obtained during this process were subsequently concentrated and purified by column chromatography (Amicon Division, W.R. Grace & Co., Danvers, MA, USA). Final concentrations of rAAV/TGF-β1 Mut and Wt were in the order of 10⁶ infectious particle/ml.

### Titering rAAV

An infectious center assay was done to determine the infectious titer of each rAAV stock. Hela cells were plated in a 35-mm -well plate to an 80 % confluence. Ad5 and wild type AAV were added to the cells with serial dilutions of rAAV viral particle stocks. 24 hours later, the cells were lysed in situ and their DNA fixed on nitrocellulose membranes using a colony lift assay. The membranes were hybridized with ³²P-labeled TGF-β1 DNA probe (southern blot) and then exposed to autoradiography.

### Infection procedures

rAAV amplified stocks were purified as described above. OVCAR or HeLa cells were plated and grown in a 96 wells plate (or a 16 wells chamber slide) at 50-80% confluence. rAAV/TGF-β1 Wt, rAAV/TGF-β1Mut or rAAV/GFP vectors were diluted in a 2% FCS containing medium along with AdM (Ad5del308δTP), in equal ratios. The virus containing media were added onto the cells for 2 hours at 37 °C. Afterwards the media were changed back to 10% and 20% FCS for HeLa and OVCAR respectively. Three days after infection, cells were either fixed for immunostaining or trypsinized for direct counting under a light microscope using a hematocytometer.

### Immunocytochemistry

Cells were grown in 16-well chamber slides and either transfected or infected as described above. After 72 hours cells were fixed with 4% formaldehyde for 10 min. and then washed thoroughly with PBS (3X 3 min.). Cells were subsequently treated with 0.1% H₂O₂ in methanol then washed again; following this was an incubation for 20 min. in Normal Goat Serum. The TGFβ1 pAb (Promega) was 1/200 times diluted in PBS and added onto the cells for an overnight incubation at 4 °C. The following day, cells were washed with PBS, and the secondary goat anti-rabbit antibody diluted 1/300 times was added for 30 minutes. Washing and then 30 minutes incubation in StreptABComplex, biotinylated horseradish peroxidase, followed this. After washing, addition of diaminobenzidine / H₂O₂ permitted the visualization of the immunocomplexes. Finally hematoxylin served as a nuclear counterstain.

### ELISA

Supernatants of either transfected or infected cells were collected after 3 days of incubation and tested for TGF-β1 content using Promega's TGF-β1 Emax ImmunoAssay system. In brief, titer plates were coated with TGF-β1 mAb in carbonate buffer for 18 hours at 4 °C. After blocking the unspecific binding sites; the supernatants were added into the wells (dilutions1/30 or 1/50). TGF-β1 standard was added in a 1/2 serial dilution. Incubation time was 1,5 hours at 37 °C. After washing, the wells were immersed for 2 hours at 37 °C in rabbit anti TGF-β1 pAb. After binding of the third antibody (goat anti-rabbit IgG, peroxidase-conjugate), the substrate TMB/ H₂O₂ was added and the color reaction developed. The reaction was stopped by addition of 1 M phosphoric acid. Measurements were done in an ELISA multiwell reader at 450 nm. Sample buffer and fresh media were used as blanks. In order to measure the total (latent and active) TGF-β1 fraction, the supernatants were first acid activated by addition of HCl to a concentration of 20 mM for 15 min. and then neutralized by addition of NaOH (20mM final concentration).

### Results

### Transfection of 293 cells with TGF-β1 wild type and mutant constructs.

Recombinant AAV/TGF-β1 Wt and AAV/TGF-β1 Mut expression plasmids were used to transfect embryonic kidney 293 cells. Transfection efficiency was about 50 % as assessed with the rAAV/GFP plasmid. Expression of the TGF-β1 mRNA was detected after transfection. The untransfected cells have almost no detectable endogenous TGF-β1 mRNA. Similarly no detectable mRNA was found in cells transfected with a non-TGF-β1 coding rAAV plasmid or mock transfected 293 cells. (Fig. 1 d.)

In the same cells the presence of TGF-β1 protein was verified using immunohistochemistry. Cells were fixed and immunostained using a pAb against TGF-β1. The immunostaining shows a clear expression of the protein in transfected cells (Fig. 2 a, b). TGF-β1 Wt (data not shown) and TGF-β1 Mut transfected cells (fig 2) express the recombinant protein at the same intensity. Background staining was negligible.

### Proliferation inhibitory effect of TGF-β1 transgene expression on ovarian cancer cells.

OVCAR-3 and AZ364 (TGF-β1 sensitive) cells were also transfected with rAAV plasmid construct. In contrast to 293 cells, transfection efficiencies using lipofectamine in both cell lines were low (3-5%). Despite this a growth inhibitory effect could be detected. In AZ364 cells, only with the rAAV/TGF-β1 Mut a 45% growth inhibition was observed after 7 days (when AZ364 mock-transfected cells reached confluence). In OVCAR-3 cells both rAAV/TGF-β1 mutant and wild type induced a suppression of proliferation at day 3 (when OVCAR-3 mock-transfected cells reached confluence). Again the effect was more pronounced with rAAV/TGF-β1 Mut (30% inhibition) than with the wild type TGF-β1 (7 % inhibition). No significant difference was found between GFP construct transfected cells and mock transfected cells. (Fig. 3 a, b)

Secretion of TGF-β1 by transfected 293 and ovarian cancer cells

The magnitude of the growth inhibition found exceeded what could be expected from the low transfection efficiencies and supported the concept of a possible paracrine effect. Therefore the presence of TGF-β1 in the media of transfected ovarian cancer cell lines was determined by the ability of conditioned media to inhibit growth of sensitive CCL-64 cells and the amount of TGF-β1 (active and latent) secreted was assayed with an ELISA method.

Conditioned media of the transfected 293 cells were tested for proliferation inhibitory activity. Medium collected from the 293 cells expressing the mutant TGF-β1 mRNA induce a strong proliferation inhibition, even cell death, of the mink lung epithelial cells CCL-64 (TGF-β1 sensitive). However, the medium obtained from the 293 cells transfected with a plasmid bearing the wild type TGF-β1 construct showed no significant proliferation inhibitory effect. (Fig. 4 a)

OVCAR cells were transfected with rAAV plasmid constructs and after 3 days the conditioned media were transferred onto CCL-64. A considerable growth inhibition was observed in CCL-64 cells incubated in supernatant from rAAV/TGF-β1 Mut plasmid transfected OVCAR. (Fig 4 b)

CCL-64 cells were not significantly growth inhibited by supernatant from OVCAR-3 cells transfected with either TGF-β1 Wt or GFP plasmids.

Table 1 represents the concentration of TGF-β1 in the conditioned media of TGF-β1 plasmid construct transfected 293 cells and rAAV/TGF-β1 viral vector infected OVCAR-3 cells. (1) Data obtained from supernatants without acid activation represent the bioactive fraction. (2) Data obtained from supernatants with an additional acid activation represent the total amount of secreted TGF-β1. Each determination is an average of 3 different assays.

**Table 1.**

| active TGF-b | rAAV/Mut | rAAV/Wt | rAAV/GFP | Mock |
|---|---|---|---|---|
| 293 cells | 26. ng/ml | < 1 ng/ml | < 1 ng/ml | < 1 ng/ml |
| OVCAR cells | 5 ng/nl | 1.5 ng/ml | < 1 ng/ml | < 1 ng/ml |
| 1) Amounts of bioactive TGF-betal in conditioned media without acid activation | | | | |
| latent +active | rAAV/Mut | rAAV/Wt | rAAV/GFP | Mock |
| 293 cells | 153 ng/ml | 45 ng/ml | < 1 ng/ml | < 1 ng/ml |
| OVCAR cells | 10 ng/ml | 1.6 ng/ml | 1 ng/ml | < 1 ng/ml |
| 2) Amounts of TGF-betal (latent +bioactive) in conditioned media with acid activation. | | | | |

### rAAV/TGF-β1 infected OVCAR cells express TGF-β1 protein

OVCAR and HeLa cells were infected with the rAAV/TGF-β1 viral vectors at an MOI of 1 (and AdM as a helper for enhanced expression at an MOI of 1). Three days after infection the cells were fixed and immunostained. TGF-β1 expression was found in both HeLa and OVCAR cells (expression percentage 10-15 %). Despite the fact that a MOI of 1 of rAAV/TGF-β1 particles was used, only a low percentage of the cells did express the protein even with the help of an AdM that is supposed to increase expression of the protein.
(Fig. 2 c, d and e, f).

### Proliferation inhibitory effect of rAAV/TGF-β1 infection of OVCAR cells.

OVCAR cells were infected with the rAAV/TGF-β1 viral vectors and AdM (MOI 1) as a helper. AAV required the help of adenovirus not. only in the process of replication but also for expressing the viral genes or the transgene in the case of rAAV. An adenovirus protein (E4ORF6) helps convert single stranded (ss) rAAV DNA into double stranded (ds) that can code for a protein. An adenovirus mutant (designated: AdM) lacking the preterminal protein provides such helper functions without being able to replicate itself (16). This AdM was used then to co-infect cells in culture.

A complete growth inhibition was observed after infection of OVCAR cells with the rAAV/TGF-β1 Mut vector but also by the rAAV/TGF-β1 Wt and rAAV/GFP vectors, and to a lesser extent viral AdM alone (Fig. 5 a). Growth inhibition by rAAV/GFP particles was pronounced but significantly less than with both TGF-β1 particles. Therefore a specific effect could be attributed to TGF-β1. An additional effect could be attributed to the cytopathic properties of the added AdM and, as previously shown by others and us, the wild type AAV particles present in all rAAV preparations might also negatively influence cell growth. When we increased AdM up to an MOI of 10, a complete (100%) cell death was observed.

### Secretion of TGF-β1 by rAAV/TGF-β1 infected OVCAR cells

To test the secretion of TGF-β1 by rAAV infected OVCAR cells, we transferred the conditioned media onto sensitive CCL-64 cells. Growth inhibitory activity was observed in conditioned medium from rAAV/TGF-β1 Mut infected cells (Fig 5 b). The supernatants from rAAV/TGF-β1 Wt and rAAV/GFP infected cells had a lesser growth inhibitory effect and contained less TGF-β1 as measured by ELISA. OVCAR cells infected with the rAAV/TGF-β Mut viruses showed a remarkable secretion of the TGF-β1 peptide. The amounts were about 5 ng/ml. (see table 1)

## Claims

1. A recombinant adeno-associated viral construct comprising at least:
- a first terminal repeat of an Adeno Associated Virus
- a strong heterologous promoter
- an heterologous DNA with at least 90% homology to the gene encoding for a *constitutively activated TGF-β1 peptide,* said gene being under the control of said promoter
- a polyadenylation signal
- a second terminal repeat of an Adeno Associated Virus.

2. The recombinant adeno-associated viral construct of claim 1 wherein said adeno-viral construct is comprised in a plasmid and/or a recombinant viral particle.

3. The recombinant adeno-associated viral construct of claim 1 or 2, wherein the TGF-β1 peptide is the TGF-β1 peptide present in LMBP 4281.

4. The recombinant adeno-associated viral construct of any of the claims 1 to 3 further comprising nucleotidic sequences encoding suitable regulatory elements so as to effect expression of the *constitutively activated TGF-β1 peptide* in a suitable host cell.

5. A host cell genetically transformed by the construct according to any of the claims 1 to 4.

6. A host cell according to claim 5, **characterized in that** said host cell is a human tumor cell.

7. A pharmaceutical composition comprising the recombinant adeno-associated viral construct of any of the claims 1 to 4 or the cell according to claim 5 or 6 and a pharmaceutically acceptable carrier.

8. The pharmaceutical composition of claim 7 wherein the recombinant adeno-associated viral construct is comprised in a recombinant viral particle.

9. A method for inhibiting the proliferation of cells, comprising at least the step of transferring a sufficient amount of the recombinant adeno-associated viral construct according to any one of the claims 1 to 4 into said cells.

10. The method of claim 9, **characterized in that** said cells are epithelial cells.

11. The method according to claim 10, **characterized in that** said epithelial cells are mammary epithelial cells, ovarian epithelial cells or lung epithelial cells.

12. The method according to claim 10 or 11, **characterized in that** said cells are cancer cells.

13. The method according to claim 12, **characterized in that** said cancer cells are selected from the group consisting of human melanoma cells, human mammary tumor cells, human ovarian tumor cells , lung tumor cells, human sarcoma cells and carcinoma cells.

14. Use of a sufficient amount of the pharmaceutical composition according to claim 7 or 8 for the preparation of a medicament in the treatment and/or the prevention of cancers.

15. Non- human animal, genetically modified by the recombinant adeno-associated viral construct according to any of the claims 1 to 4.


SEQUENCE LISTING

<110>  Vrije Universiteit Brussel

<120>  RECOMBINANT VECTORS DERIVED FROM ADENO-ASSOCIATED VIRUS FOR GENE THERAPY

<130>  P.VUB.08/EP

<160>  5

<170>  PatentIn version 3.1

<210>  1
<211>  29
<212>  DNA
<213>  Artificial Sequence

<220>
<223>  Primer for PCR

<400>  1
atatgcggcc gcctgttcgc gctctcgga                                        29


<210>  2
<211>  28
<212>  DNA
<213>  Artificial Sequence

<220>
<223>  Primer for PCR

<400>  2
atatgtcgac tcagctgcac ttgcagga                                         28


<210>  3
<211>  29
<212>  DNA
<213>  Artificial Sequence

<220>
<223>  mutagenic oligonucleotide

<400>  3
ccttagcgcc cacagctcct ctgacagca                                        29


<210>  4
<211>  17
<212>  DNA
<213>  TGF-beta1Mut

<400>  4
cccacagctc ctctgac                                                     17


<210>  5
<211>  17
<212>  DNA
<213>  TGF-beta1Wt

<400> 5
cccactgctg gtgtgac 17